(19) Europäisches Patentamt / European Patent Office / Office européen des brevets

(11) EP 2 922 856 B1

(12) # FASCICULE DE BREVET EUROPEEN

(45) Date de publication et mention de la délivrance du brevet:
**07.12.2016 Bulletin 2016/49**

(21) Numéro de dépôt: **13808114.6**

(22) Date de dépôt: **26.11.2013**

(51) Int Cl.:
***C07D 493/04*** (2006.01) ***C08K 5/15*** (2006.01)

(86) Numéro de dépôt international:
**PCT/FR2013/052866**

(87) Numéro de publication internationale:
**WO 2014/080152 (30.05.2014 Gazette 2014/22)**

(54) **PROCEDE DE FABRICATION DE COMPOSITIONS COMPLEXES DE DIESTERS MIXTES DE 1,4 : 3,6-DIANHYDROHEXITOL**

VERFAHREN ZUR HERSTELLUNG KOMPLEXER ZUSAMMENSETZUNGEN AUS GEMISCHTEN DIESTERN VON 1,4: 3,6-DIANHYDROHEXITOL

METHOD FOR PRODUCING COMPLEX COMPOSITIONS OF MIXED DIESTERS OF 1,4: 3,6-DIANHYDROHEXITOL

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **26.11.2012 FR 1261253**

(43) Date de publication de la demande:
**30.09.2015 Bulletin 2015/40**

(73) Titulaire: **Roquette Frères**
**62136 Lestrem (FR)**

(72) Inventeurs:
- **FERON, Thierry**
  **F-62232 Fouquieres Les Bethune (FR)**
- **VERRAES, Arnaud**
  **F-59136 Wavrin (FR)**
- **WYART, Hervé**
  **F-62149 Cuinchy (FR)**
- **BAURAIN, Catherine**
  **F-62400 Locon (FR)**
- **BROCARD, Juliette**
  **F-62136 Lestrem (FR)**
- **FUERTES, Patrick**
  **F-59160 Lomme (FR)**

(74) Mandataire: **Cabinet Plasseraud**
**66, rue de la Chaussée d'Antin**
**75440 Paris Cedex 09 (FR)**

(56) Documents cités:
**WO-A1-99/45060**
**WO-A1-2008/095571**
**WO-A1-2013/092649**
**WO-A1-2013/092655**
**US-A- 2 387 842**

- **HACHIHAMA Y ET AL: "Preparation of plasticizers from carbohydrate sources. I. Levulinic acid esters. II. Sorbide esters", TECHNOLOGY REPORTS OF THE OSAKA UNIVERSITY, OSAKA, JP, vol. 3, no. 72, 1 janvier 1953 (1953-01-01), pages 191-200, XP008089789,**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 2 922 856 B1

## Description

### Domaine de l'invention

L'invention a pour objet un procédé de fabrication d'une composition comprenant des mélanges complexes d'esters de 1,4 :3,6 dianhydrohexitol. Un second objet de l'invention porte également sur ladite composition. Un autre aspect de l'invention porte sur l'utilisation de cette composition pour la plastification de polymères.

### Etat de la technique

Du fait de leurs nombreux avantages, l'utilisation de polymères synthétiques s'est généralisée dans de nombreuses applications depuis le siècle dernier.

Cependant, ces polymères peuvent présenter des inconvénients, comme en particulier leurs propriétés mécaniques qui peuvent être insuffisantes pour certaines utilisations. Par exemple, ils peuvent présenter un allongement à la rupture très faible à température ambiante ou être peu résistants au choc.

De plus, il peut être nécessaire de modifier le comportement en phase fondue de ces polymères, notamment pour pouvoir les mettre en oeuvre dans des procédés de transformation de type enduction ou encore en calandrage. En d'autres termes, il est nécessaire que le polymère ait dépassé sa température de fusion, ou encore sa température de gélification, et qu'ainsi le polymère présente, dans cet état gélifié, une viscosité adaptée au procédé de mise en forme pour pouvoir être correctement transformé.

Pour pouvoir les utiliser dans des applications plus variées, il est également nécessaire de modifier les propriétés de ces polymères, par exemple pour les rendre plus souples, plus résistants aux chocs ou encore leur permettre d'avoir un aspect plus doux.

Pour ceci, ces polymères peuvent être mélangés avec des « plastifiants ».

Par « plastifiant », on entend tout produit qui, lorsqu'il est mélangé en quantité suffisante avec un polymère, a pour fonction de diminuer la température de transition vitreuse dudit polymère.

En diminuant la température de transition vitreuse du polymère, la souplesse de ce dernier est augmentée et les propriétés mécaniques de ce polymère plastifié sont modifiées. Ainsi, en ajoutant un plastifiant à une composition de polymère, on observe généralement une diminution du module, notamment du module à 100% d'allongement, une diminution de la contrainte à la rupture et/ou une augmentation de la déformation à la rupture.

Ces propriétés modifiées du polymère lui permettent alors d'être utilisé dans des applications plus variées, par exemple dans des films ou des feuilles souples.

Lors du procédé de mise en oeuvre de la matière plastique, les plastifiants sont généralement mélangés au polymère, ce qui permet la diminution de la température de ramollissement du polymère.

Ce mélange peut se faire par différents procédés de mise en oeuvre.

Dans le cas des polychlorures de vinyle (PVC) par exemple, le polymère peut être transformé en objet par différentes techniques de transformation des matériaux thermoplastiques, et en particulier par extrusion, par calandrage ou encore par enduction via un procédé de type plastisol.

Afin d'obtenir ce mélange thermoplastique, on mélange le PVC avec le plastifiant en apportant à ce système de l'énergie, sous forme de température et d'énergie mécanique. Dans le cas de l'extrusion, ce mélange se fait dans un système fermé. Dans le cas d'un mélange sur cylindres, ce mélange se fait dans un système ouvert. Le polymère peut être ensuite mis en forme, par exemple par des procédés de thermoformage ou de calandrage. Généralement, on réalise une étape de mélange à sec (en anglais *dry blend*) avant l'étape de mélange thermomécanique.

Selon le procédé plastisol, on réalise généralement un mélange pour former une pâte de PVC, puis cette pâte est mise en forme par une étape d'enduction ou de moulage, et la pâte est ensuite chauffée dans un four pour former la pièce.

Quel que soit le procédé, il faut que le polymère soit correctement fondu ou gélifié pour pouvoir former de manière satisfaisante l'objet obtenu à la fin du procédé.

Pour des raisons de facilité de stockage, d'utilisation et de dosage du plastifiant, on utilise généralement des plastifiants liquides à température ambiante.

Pour tous ces procédés d'obtention de mélanges thermoplastiques à partir de PVC, on utilise encore très souvent des plastifiants de la famille des esters phtaliques. Il s'agit encore à ce jour très généralement de phtalate de dioctyle ou de phtalate de diisononyle. Ces plastifiants sont très efficaces pour la plastification de polymères, et sont facilement disponibles sur le marché, pour un coût relativement faible. Cependant, du fait des problèmes de toxicité des phtalates, d'autres plastifiants ont également été développés ces dernières années, comme l'acide polycarboxylique cyclohexane et ses dérivés, qui ont fait l'objet des demandes de brevet WO 00/78853 et WO 99/32427. A titre d'exemple, on peut citer l'ester de diisononyle d'acide dicarboxylique 1,2-cyclohexane (DINCH) commercialisé par BASF sous la marque Hexamoll®.

Comme autres plastifiants, on peut également citer les dérivés esters de glycérol, tel que le Grindsted® SOFT-N-SAFE obtenu à partir de glycérol et d'huile de ricin et commercialisé par la société Danisco. Ces plastifiants présentent l'avantage d'être obtenus à partir de produits biosourcés.

L'utilisation de dérivés de 1,4 :3,6 dianhydrohexitols comme plastifiants de polymères a déjà été décrite dans le document WO 99/45060. Ces dérivés ne présentent pas les problèmes de toxicité des phtalates. Ces plastifiants présentent en outre l'avantage d'être partiellement biosourcés, voire totalement biosourcés. Dans cette demande sont décrits dans les exemples les plastifiants liquides à température ambiante suivants : le dioctanoate d'isosorbide, le dibutanoate d'isosorbide, le dihexanoate d'isosorbide et de le di(2-ethylhexanoate) d'isosorbide. Ces plastifiants sont également décrits dans le document WO 2008/095571 A1, qui décrit des diesters aliphatiques à 9 atomes de carbone. Le document *Preparation of plasticizers from carbohydrate sources. I. Levulinic acid esters. II. Sorbide esters,* (Hachihama et al., Technology reports of the Osaka University, Vol. 3, n°72, 1953, pages 191-200) décrit quant à lui des esters aliphatiques à 8 atomes de carbone ainsi que des esters aliphatiques à 10 atomes de carbone. Le brevet US 2387842 A décrit quant à lui des diesters aliphatiques mixtes, ceux-ci étant également utiles comme plastifiants.

WO 2013/092655 et WO 2013/092649 divulguent des mélanges d'esters, en particulier d'ester de sorbitol avec des mélanges d'acides gras en C8/C10; ainsi que leur synthèse et utilisation pour plastifier des polymères.

Les propriétés mécaniques des polymères plastifiés avec ces dérivés sont excellentes, proches de celles obtenues avec les plastifiants de type phtalates. Dans le cadre de ses recherches, la Société Demanderesse a cependant constaté que ces composés, et en particulier le dioctanoate d'isosorbide, avaient tendance à « migrer » hors de l'objet formé à partir de la matière plastique. On peut également observer par exemple que le plastifiant « exsude ». Or, la migration du plastifiant peut avoir comme conséquences une perte au cours du temps de propriétés mécaniques et/ou optiques du polymère, une dégradation de l'aspect et du toucher, voire poser des problèmes de défaut d'impression lorsque l'on souhaite imprimer dans un second temps la surface du polymère. Pour certaines applications, la résistance à la lixiviation - c'est-à-dire la migration dans un bain d'eau chaude - peut également être une propriété déterminante, notamment par exemple pour la fabrication de géomembranes. Par ailleurs, certains de ces composés présentent un autre problème : ils peuvent présenter une volatilité encore un peu élevée. Celle-ci peut être gênante, en particulier au moment où le transformateur mélange le plastifiant avec le polymère, et plus encore lorsqu'il utilise un système de mélange ouvert. En effet, il y a une légère perte de plastifiant lors de la transformation.

Il reste donc un besoin de trouver de nouvelles compositions plastifiantes, liquides à température ambiante, ces compositions permettant de résoudre les problèmes précédemment cités et en particulier de limiter les phénomènes de migration observés dans les polymères plastifiés avec lesdites compositions.

## Résumé de l'invention

C'est justement le mérite de la Demanderesse qui est parvenue à trouver une composition particulière de diesters de dianhydrohexitol permettant de plastifier de manière très efficace les polymères, tout en limitant les problèmes de migration précédemment décrits.

Cette composition de diesters de 1,4 : 3,6-dianhydrohexitol est susceptible d'être obtenue par un procédé de fabrication, objet de la présente invention, ledit procédé comprenant une étape d'estérification de 1,4 : 3,6-dianhydrohexitol par une composition d'acides carboxyliques, ladite composition d'acides carboxyliques comprenant :

- au moins un acide de formule C7H15COOH (X) et un acide de formule C9H19COOH (Y), le ratio massique (X)/(Y) allant de 10/90 à 90/10 ;
- au moins un acide de formule différente de (X) et (Y) choisi parmi l'acide hexanoïque, l'acide laurique, l'acide myristique, l'acide oléïque ou l'acide linoléique.

Comme montré dans les exemples, la composition selon l'invention présente l'avantage de pouvoir être stockée facilement et être peu volatile par rapport à des compositions obtenues à partir de compositions d'acides carboxyliques exemptes d'acide de formule différente de (X) et (Y) choisi parmi l'acide hexanoïque, l'acide laurique, l'acide myristique, l'acide oléïque ou l'acide linoléique.

De plus, contrairement aux décanoates de 1,4 : 3,6-dianhydrohexitol par exemple, cette composition présente également l'avantage de pouvoir être liquide à température ambiante, ce qui permet un dosage aisé du plastifiant lors de la fabrication des objets polymériques plastifiés à l'aide de cette composition.

La composition présente également l'avantage de pouvoir être fabriquée à partir d'une composition d'acides carboxyliques moins coûteux que ceux utilisés pour la fabrication de certains diesters de 1,4 : 3,6-dianhydrohexitol déjà connus de la demande WO 99/45060, notamment les dioctanoates de 1,4 : 3,6-dianhydrohexitol qui sont fabriqués exclusivement à partir d'acide n-octanoïque comme acide carboxylique.

## Description détaillée de l'invention

Le procédé selon l'invention comporte une étape d'estérification de 1,4 : 3,6-dianhydrohexitol avec une composition d'acides carboxyliques.

Avant de réaliser l'étape d'estérification à proprement parler, le procédé selon l'invention comprend généralement une étape d'introduction de 1,4 : 3,6-dianhydrohexitol et une étape d'introduction d'acides carboxyliques ou d'une composition d'acides carboxyliques dans un réacteur.

Le 1,4 : 3,6-dianhydrohexitol utilisé dans le procédé selon l'invention peut être choisi parmi l'isomannide, l'isoidide, l'isosorbide et leurs mélanges, préférentiellement l'isosorbide. Les diesters ainsi fabriqués à partir du procédé selon l'invention sont alors respectivement des diesters d'isomannide, d'isoidide, d'isosorbide ou des mélanges de ces diesters.

La composition d'acides carboxyliques utile au procédé selon l'invention comprend au moins deux acides carboxyliques de formule $C7H15COOH$ (X) et de formule $C9H19COOH$ (Y) et un ou plusieurs acides carboxyliques différents de (X) et (Y), choisi parmi l'acide hexanoïque, l'acide laurique, l'acide myristique, l'acide oléïque ou l'acide linoléique.

Les deux acides X et Y peuvent être, dépendamment ou indépendamment l'un de l'autre, linéaires ou ramifiés, et sont préférentiellement linéaires.

L'acide carboxylique différent de (X) et (Y) est préférentiellement l'acide laurique.

La composition d'acides carboxyliques utilisée dans le procédé selon l'invention peut comprendre, par rapport à sa masse totale, au moins 50% en masse d'acides (X) et (Y), par exemple au moins 65%, au moins 80%, par exemple au moins 90%.

La composition d'acides carboxyliques utilisée dans le procédé selon l'invention peut comprendre, par rapport à sa masse totale, au plus 90% en masse d'acides (X) et (Y), par exemple au plus 80%.

Avantageusement, le ratio massique (X)/(Y) va de 15/85 à 75/25.

De préférence, le ratio massique (X)/(Y) va de 20/80 à 65/35.

Préférentiellement, le ratio massique (X)/(Y) va de 25/75 à 55/45.

Selon une variante, le ratio massique (X)/(Y) va de 25/75 à 45/55, par exemple de 26/74 à 34/66.

Selon une autre variante, le ratio massique (X)/(Y) va de 25/75 à 75/25, avantageusement de 26/74 à 65/35.

Selon encore une autre variante, le ratio massique (X)/(Y) va de 45/55 à 75/25, avantageusement de 55/45 à 65/35.

Selon un mode de réalisation, le ratio molaire (X)/(Y) est différent de 25/75, et/ou de 40/60, et/ou de 45/55, et/ou de 50/50, et/ou de 57/43, et/ou de 58/42, et/ou de 65/35, et/ou de 75/25 et/ou de 80/20 et/ou de 85/15.

Selon un mode de réalisation, le ratio molaire (X)/(Y) n'est pas compris dans les gammes suivantes : 45/55 à 85/15, 45/55 à 75/25, 45/55 à 65/35 ou 50/50 à 65/35.

L'étape d'estérification du procédé selon l'invention peut être réalisée par toute méthode connue d'estérification de 1,4 : 3,6-dianhydrohexitol par un acide carboxylique, ledit procédé différant des procédés de l'art antérieur en ce que l'on utilise, en lieu et place de l'acide carboxylique classiquement utilisé, une composition d'acides carboxyliques comprenant au moins un acide (X) et un acide (Y), le ratio massique (X)/(Y) allant de 10/90 à 90/10 et un ou plusieurs acides carboxyliques différents de (X) et (Y), choisis parmi l'acide hexanoïque, l'acide laurique, l'acide myristique, l'acide oléïque ou l'acide linoléique.

En faisant varier, le ratio massique des acides gras, on fait varier les quantités de diesters formées lors de cette étape comme décrit par la suite.

La réaction d'estérification peut être réalisée dans les conditions classiques de mise en oeuvre déjà utilisées dans la littérature. Ces méthodes d'estérification sont décrites par exemple dans les documents WO 99/45060 A1 ou WO 2006/103338 A1.

Pour former la composition de diesters, on fait généralement réagir 2 moles d'acides carboxyliques par mole de 1,4 : 3,6-dianhydrohexitol dans le réacteur d'estérification.

La somme totale de moles d'acides carboxyliques (X), (Y) et d'autres acides carboxyliques éventuels introduits dans le réacteur va avantageusement de 2 à 20 pour 1 mole de 1,4 : 3,6-dianhydrohexitol, de préférence de 2,5 à 5, tout préférentiellement de 2,8 à 3,5.

On peut réaliser l'étape d'estérification en présence d'au moins un catalyseur acide. Le catalyseur acide mis en oeuvre en vue de l'estérification peut être de nature très variée, par exemple peut être un acide choisi parmi l'acide hypophosphoreux, l'acide chlorhydrique, l'acide sulfurique, l'acide para-toluène sulfonique (APTS), l'acide méthanesulfonique (AMS), l'acide trifluorométhanesulfonique, l'acide trifluoroacétique, l'acide trichloroanétique, l'éthyl-2-hexanoate d'étain, l'acide phosphotungstique et l'acide silicotungstique ou un mélange de ces acides ou une résine macroporeuse ou non macroporeuse comprenant au moins un de ces acides. De préférence, le catalyseur comprend de l'acide hypophosphoreux.

Dans le cas de mélanges de catalyseurs, ils peuvent être introduits dans le milieu réactionnel simultanément,

ou non.

La quantité massique de catalyseur acide peut aller de 0,05 à 20% par rapport à la masse de 1,4 : 3,6-dianhydrohexitol introduite dans le réacteur, par exemple de 0,1 à 10%.

La température dans le réacteur peut aller de 90 à 200°C, généralement de 100 à 160°C. Pour réaliser la réaction d'estérification, on élimine généralement l'eau afin de permettre la formation du diester, cette élimination pouvant se faire par exemple par distillation du milieu réactionnel. Afin de faciliter cette élimination, on peut placer le milieu réactionnel sous vide, par exemple à un niveau allant de 10 à 200 mbar. On peut faire varier les conditions réactionnelles telles que le niveau de vide et la température lors de la réaction.

La réaction d'estérification dure généralement le temps d'obtenir une conversion satisfaisante en diester de 1,4 : 3,6-dianhydrohexitol. Elle peut varier largement et aller de 1 à 10 heures.

On peut également réaliser une étape de neutralisation du catalyseur introduit, en introduisant une base, par exemple de la soude, dans des quantités molaires équivalentes aux quantités molaires de catalyseur introduit.

Le procédé de fabrication peut comprendre en outre une étape de purification de la composition issue de l'étape d'estérification. Celle-ci consiste avantageusement en au moins une étape d'évaporation, par exemple par distillation, permettant d'éliminer la plus grande partie ou la quasi-totalité de l'acide carboxylique encore présent à l'issue de l'étape d'estérification. Lors de cette étape, la composition de diester peut être soumise à des conditions de température comprises entre 100 et 250°C et à des pressions réduites comprises entre 0,1 et 50 mbar. De préférence, cette étape se fait dans un évaporateur continu. Un tel évaporateur, par exemple de type « à flot tombant » ou mieux, de type à film raclé ou « *short path »,* permet de limiter les températures et temps de séjour auxquels est soumise la composition issue de l'étape d'estérification.

Le procédé peut également comprendre une étape de décoloration de la composition de diester, par exemple en utilisant des charbons actifs ou de l'eau oxygénée. Le traitement par du charbon actif se fait par exemple par mise en contact de la composition avec de 1 à 3 % en poids de charbon actif. La température lors de ce traitement peut être à voisine de 100°C. La durée est généralement de plusieurs dizaines de minutes, par exemple pendant environ une heure. A la fin du traitement, le charbon actif est séparé par filtration. Un traitement classique de décoloration par eau oxygénée consiste par exemple à introduire dans la composition à décolorer, sur une période allant par exemple de 30 à 60 minutes, de 0,5 à 2 % d'eau oxygénée à 100 %, à une température comprise entre 90°C et 100°C, puis on agite la composition pendant une à deux heures à cette température. Lorsque l'on souhaite associer ces deux types de traitement de décoloration, le traitement à l'eau oxygénée précède de préférence celui par le charbon actif. Ce dernier permet en effet de détruire les peroxydes éventuellement présents.

Comme expliqué précédemment, un autre objet de l'invention porte sur une composition de diesters de 1,4 : 3,6-dianhydrohexitols susceptible d'être obtenue par le procédé de fabrication décrit précédemment.

Cette composition de diesters complexe étant obtenue à partir d'une composition d'acides carboxyliques comprenant, en plus des acides (X) et (Y), un, voire plusieurs autres acides carboxyliques, ne peut être définie de manière plus satisfaisante que par son procédé de fabrication.

En effet, lorsque cette composition d'acides carboxyliques comprend un ou plusieurs autres acides carboxyliques, on obtient des compositions très complexes et diverses, qui vont dépendre de la nature et de la quantité des acides carboxyliques autres que les acides (X) et (Y) utilisés.

A titre d'exemple, lorsqu'on réalise le procédé selon l'invention à partir d'une composition d'acides carboxyliques constituée de (X), de (Y) et d'acide laurique, dans lequel le rapport massique (X)/(Y) est égal à 60/40 et dans lequel la composition d'acides carboxyliques comprend, par rapport à sa masse totale, 70% en masse du mélange (X) et (Y), la composition produite comprend en masse :

- Environ 16% de diester de formule :

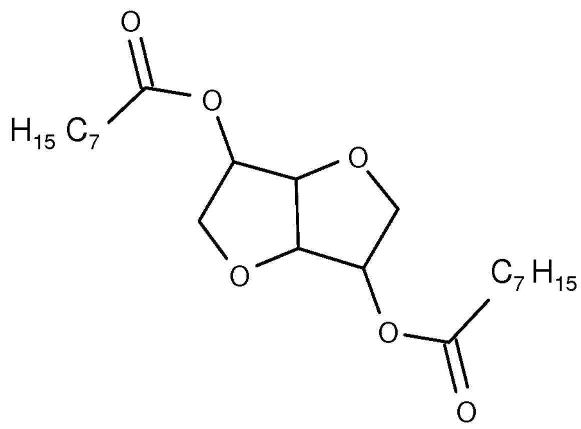

;

- Environ 24% de diester de formule :

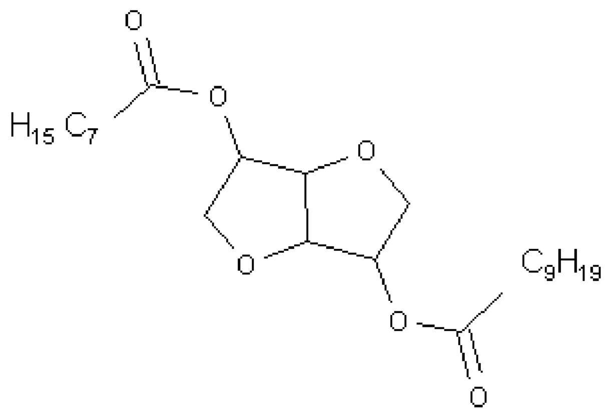

;

- Environ 24% de diester de formule :

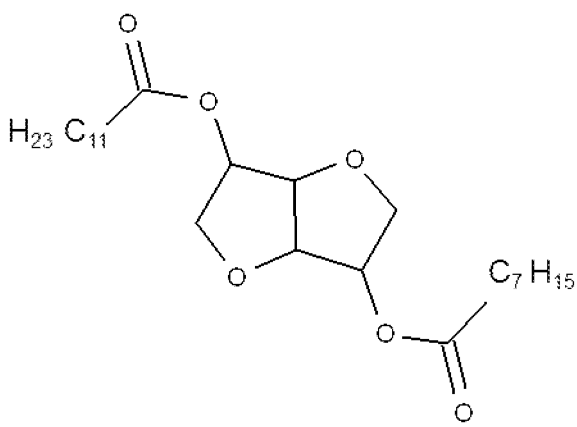

;

- Environ 9% de diester de formule :

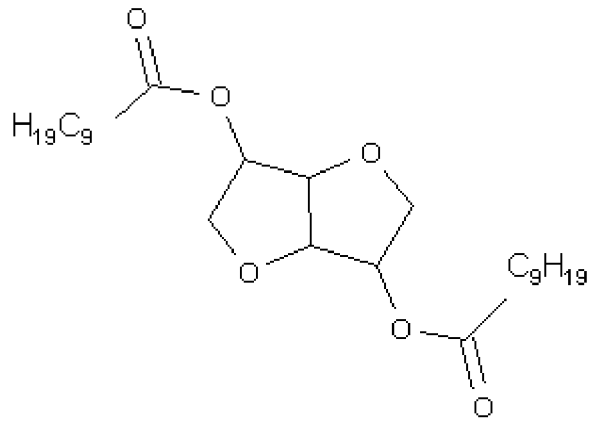

;

- Environ 18% de diester de formule :

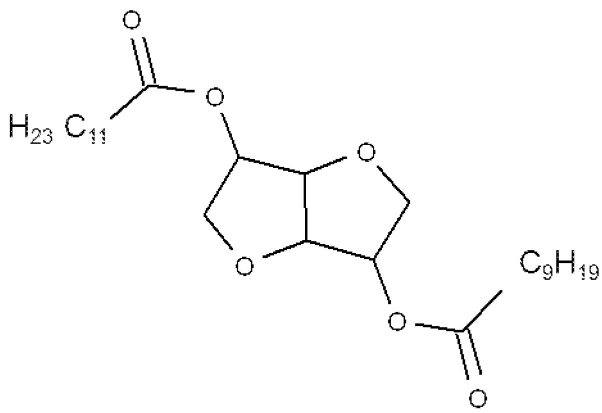

;

- Environ 9% de diester de formule :

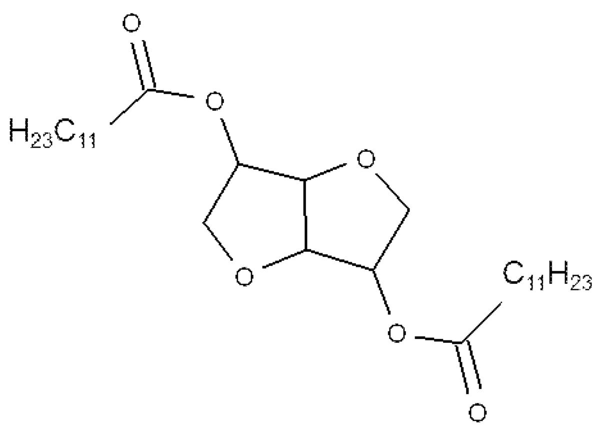

L'homme du métier pourra ainsi fabriquer les compositions selon l'invention ainsi que ses variantes préférées en utilisant le procédé décrit précédemment et en faisant varier le rapport massique (X)/(Y) et les quantités en acide carboxylique additionnel. D'autres exemples de compositions de diesters qui peuvent être produites selon le procédé de l'invention figurent également dans les exemples de la présente demande.

Les quantités massiques en différents diesters peuvent être déterminées par chromatographie phase gazeuse. Il est possible par exemple de se référer à la méthode analytique détaillée qui figure dans les exemples, la répartition massique en diester étant donnée par le ratio des aires de diester sur la somme des aires.

La composition pouvant être obtenue par le procédé selon l'invention déjà décrit comprend généralement les

espèces résiduelles suivantes, parfois à l'état de traces : des monoesters de 1,4 : 3,6-dianhydrohexitol, des acides résiduels, voire de l'eau.

La composition selon l'invention peut ainsi présenter par rapport à sa masse totale au moins 80% en masse de diester, par exemple au moins 90%, préférentiellement au moins 95%, tout préférentiellement au moins 98%.

De préférence, au moins un des diesters de la composition est un diester d'isosorbide. De préférence, la composition de diesters est une composition de diesters d'isosorbide.

Selon une variante de la composition selon l'invention, elle contient au moins 90% de carbone issu de matière renouvelable selon la norme ASTM D6866.

Comme le 1,4 : 3,6-dianhydrohexitol peut être obtenu à partir du mannitol, de l'iditol et du sorbitol, qui sont eux-mêmes obtenus à partir d'amidon, les esters de 1,4 : 3,6-dianhydrohexitol utiles à l'invention présentent en outre l'avantage de pouvoir être partiellement biosourcés, voire totalement biosourcés si on utilise des compositions d'acide carboxyliques également totalement biosourcées.

Comme décrit précédemment, la composition selon l'invention, qui peut présenter l'avantage d'être liquide à température ambiante (25°C), est particulièrement utile pour la plastification de polymères.

La Demanderesse a pu constater que la composition plastifiante permettait de plastifier les polymères de manière excellente, de façon au moins aussi satisfaisante qu'avec les dioctanoates de 1,4 : 3,6-dianhydrohexitol. De plus, toujours par rapport aux polymères plastifiés à l'aide de ces esters d'isosorbide obtenus à partir de mélanges d'acides carboxyliques (X) et (Y) exempts d'acide carboxylique additionnel, le phénomène de migration de la composition plastifiante hors du polymère est drastiquement diminué.

Le polymère peut être choisi parmi les polymères vinyliques tels que le polychlorure de vinyle et les copolymères de chlorure de vinyle, les polyuréthanes, les polyesters, les polymères cellulosiques, les amidons, les polymères acryliques, les polyacétates, les caoutchoucs naturels ou synthétiques, notamment les caoutchoucs fabriqués à partir de styrène et/ou de butadiène tels que les caoutchoucs de type SBR, BR ou NBR, les polyamides les mélanges de ces polymères, préférentiellement le polychlorure de vinyle. Par polychlorure de vinyle, on entend selon la présente invention les homopolymères de chlorure de vinyle ou les copolymères comprenant du chlorure de vinyle, par exemple les copolymères acétate de vinyle/chlorure de vinyle.

Le polymère ainsi obtenu est un polymère plastifié par la composition selon l'invention. Pour l'homme du métier, cela signifie que le polymère et la composition plastifiante sont intimement mélangés. Les constituants de la composition plastifiante sont introduits entre les chaînes du polymère solide et il en résulte, après transformation, un polymère plastifié constitué d'une phase solide.

Préalablement à son mélange avec la composition plastifiante, le polymère peut être sous toute forme, par exemple sous forme de granulés ou de poudre.

On peut également fabriquer une pâte de polymère comprenant un mélange d'une poudre de polymère et de la composition selon l'invention. Cette pâte est généralement appelée *plastisol* et permet de former des objets par les procédés décrits ci-après. Préférentiellement, le diamètre moyen de particules de la poudre est compris entre 1 et 30 $\mu$m, par exemple entre 1 et 20 $\mu$m. Dans le cas du polychlorure de vinyle, on peut obtenir ce type de poudres en préparant le PVC par émulsion ou micro-suspension. Cette pâte est généralement obtenue par mélange mécanique, préférentiellement sans chauffage, de la poudre de polymère avec la composition plastifiante.

Les mélanges ainsi obtenus sont appelés plastisols qui sont, selon les quantités de polymère et de composition plastifiante, plus ou moins fluides. Classiquement, les plastisols sont préparés dans des mélangeurs rapides de type turbine, des mélangeurs planétaires ou des mélangeurs lents qui sont des mélangeurs planétaires à pales horizontales en Z.

La composition plastifiante et le polymère sont avantageusement mélangés dans des proportions massiques telles que la quantité de composition plastifiante va de 1 à 900 parts pour 100 parts de polymère, avantageusement de 5 à 180 parts, préférentiellement de 15 à 120 parts de composition plastifiante. On peut les introduire dans le système mélangeur par tout moyen adapté, telle que trémie d'alimentation, ou de façon manuelle.

Dans le cas de la pâte de polymère, on préfère que les quantités de composition plastifiante aillent de 30 à 120 parts pour 100 parts de poudre de polymère.

Dans la composition de polymère plastifié, on peut également utiliser, en plus de la composition plastifiante et du polymère, des additifs optionnels. Ces additifs peuvent être choisis parmi les stabilisants, les anti-UV, les charges, les colorants, les pigments, les agents gonflants, les émulsifiants, les abaisseurs de viscosité différents de la composition plastifiante, les épaississants, les agents de démoulage, les agents matants, les agents d'adhésion, les agents antistatiques, les agents fongicides ou les agents odoriférants. Les quantités de chaque additif sont choisies afin d'apporter les propriétés désirées lors de la mise en oeuvre du procédé ou pour l'objet finalement obtenu. Ces additifs peuvent être introduits dans la composition directement ou sous forme de mélange-maître. La quantité en additif optionnel va généralement de 1 à 600 parts pour 100 parts de polymère (C), généralement de 2 à 80 parts.

Selon une première variante du procédé de fabrication du polymère plastifié, le procédé comprend une étape de mélange thermomécanique.

Selon cette première variante, l'étape de mélange thermomécanique est réalisée dans un système mélangeur qui est un mélangeur pour thermoplastiques. Ce mélangeur peut être choisi parmi les malaxeurs, les mélangeurs BUSS, les mélangeurs à cylindres et les extrudeuses.

La composition plastifiante peut être introduite sous la forme d'un mélange-maître.

L'étape de mélange thermomécanique est réalisée à une température adaptée à la température de transformation du polymère. A titre d'exemple, la température du mélange lors du mélange thermomécanique est préférentiellement comprise entre 60 et 200°C pour un PVC.

Pour un mélange thermomécanique, on peut utiliser un polymère sous tout type de forme, notamment sous formes de granulés ou de poudre.

Selon cette première variante, on réalise avantageusement une étape préalable de mélange à sec (en anglais *dry blend*) de la composition plastifiante avec le polymère avant le mélange thermomécanique. Ce mélange à sec peut être réalisé dans un simple mélangeur mécanique, qui peut être chauffé à une température inférieure à la température de fusion ou de gélification du polymère.

L'objet peut avantageusement être mis en forme par calandrage, injection, extrusion injection, intrusion, trempage en lit fluidisé, pistolage électrostatique, moulage, rotomoulage, extrusion moulage, frittage, thermoformage, pressage, extrusion formage, extrusion gainage, extrusion soufflage. On peut également utiliser des techniques de co-extrusion pour former des objets multicouches.

Selon une seconde variante, on utilise pour former l'objet selon l'invention un procédé de type *plastisol* avec la pâte de polymère préalablement décrite.

Dans ce type de procédé, l'étape de mise en forme est généralement une étape d'enduction, de trempage, de foulardage, de pulvérisation, de coulée, de moulage, d'embouage, ou de moulage par rotation de la pâte de polymère, ce qui permet de former un objet préformé.

L'étape de chauffage du procédé est une étape de cuisson dudit objet préformé, cette étape ayant généralement lieu après l'étape de préformage (c'est le cas par exemple de l'enduction). Elle peut parfois également avoir lieu pendant l'étape de mise en forme de l'objet préformé (c'est le cas par exemple du trempage, de l'embouage ou du moulage par rotation). Cette étape de cuisson peut se faire à une température comprise entre 60 et 300°C, par exemple entre 100 et 250°C, généralement entre 140 et 220°C. Elle peut se faire sous air ou sous atmosphère contrôlée, par exemple sous atmosphère inerte.

L'étape de mise en forme de l'objet est préférentiellement une étape d'enduction de la pâte de polymère sur un support, cette enduction étant réalisée avant l'étape de cuisson dudit support enduit. L'étape d'enduction peut être réalisée sur un support textile, un voile de verre, du métal, un polymère synthétique ou un papier.

L'enduction peut être réalisée à l'aide de toute tête d'enduction, par exemple à l'aide d'une racle ou d'un cylindre.

Cette enduction est capable d'être, selon une première sous-variante, une enduction dite « enduction sur support » comme décrite ci-dessus, ou selon une seconde sous-variante, une enduction dite « enduction sans support ». Dans ce dernier cas, le support enduit peut être détaché après cuisson et le procédé comprend en outre une étape ultérieure de séparation du support pour former un film ou une feuille de polymère plastifié. Un tel support peut être en papier siliconé.

L'étape de cuisson est généralement réalisée dans un four, par exemple un four tunnel, sur un tambour gélificateur ou sous une rampe infrarouge.

On peut également former un objet comprenant la composition de polymère plastifié.

L'objet comprenant la composition de polymère plastifié peut être tout type d'objet, tel qu'un film, une feuille, un granulé, un revêtement de sol, un revêtement mural, un tissu enduit plastique, notamment un cuir artificiel, par exemple pour chaussure, pour maroquinerie ou pour ameublement, une bâche, un *liner* par exemple pour piscine, un store, un container souple, un vêtement, un produit médical, un gant, une botte, un joint, un revêtement protecteur, un mannequin pour vitrine, un jouet par exemple un ballon ou une poupée, un tube, des profilés notamment des profilés de fenêtre, des pièces automobiles telles que tableau de bord, siège, réservoir ou appuie-tête. Ces pièces peuvent être des pièces cellulaires, moussées ou meringuées, c'est-à-dire comprenant des cellules d'air. Elles peuvent également être au contraire des pièces compactes.

Un avantage des compositions selon l'invention est qu'elles permettent d'améliorer les propriétés de fogging de pièces réalisées à partir de polymère plastifié, et notamment de PVC plastifié, à l'aide de celles-ci par rapport aux pièces de polymère plastifié à l'aide de diesters de 1,4 : 3,6-dianhydrohexitol déjà connus.

Un autre avantage des compositions selon l'invention est qu'elles permettent d'améliorer les propriétés de lixiviation de pièces réalisées à partir de polymère plastifié, et notamment de PVC plastifié, à l'aide de celles-ci par rapport aux pièces de polymère plastifié à l'aide de diesters de 1,4 : 3,6-dianhydrohexitol déjà connus.

Ceci est particulièrement important pour une utilisation en automobile ou dans les transports.

Bien évidemment, des améliorations sont encore plus importantes lorsque ces compositions sont riches en diester, c'est-à-dire lorsque leur quantité en monoesters de 1,4 : 3,6-dianhydrohexitol et/ou en acides résiduels est faible puisque ces espèces sont connues pour migrer plus facilement.

L'invention va maintenant être détaillée dans les exemples de réalisation ci-après. Il est précisé que ces derniers ne limitent en rien l'objet de la présente invention.

**Exemples :**

**EXEMPLE A: Synthèse des compositions de diesters**

Méthodes analytiques

La quantité massique en diester dans la composition et les proportions massiques en chaque diester formé est mesurée par chromatographie phase gazeuse sur un appareillage de type Varian 3400 avec détection FID et injecteur split/splitless type 1077. La colonne utilisée est une DB1 de marque J & W Scientific de longueur 30 mètres, de diamètre interne 0,32 mm, d'épaisseur de film 0.25 μm. Les conditions de température sont : injecteur et détecteur : 300°C ; colonne : programmation de 100°C jusque 320°C à raison de 7°C/mn, maintien 15 mn à 320°C. L'injection se fait en split à 80 ml/mn, la pression en tête de colonne étant de 14 psi et le gaz vecteur utilisé est de l'hélium.

La quantité massique en diester est donnée par le ratio de la somme des aires des composés correspondant aux diesters d'isosorbide sur la somme des aires de la totalité des composés dont le temps de rétention est compris entre 4 et 40min.

**Exemple 1 (COMPARATIF)**

On réalise un essai comparatif (« ESSAI 1 ») selon le protocole opératoire général suivant.

Dans un réacteur en verre de 1 litre muni d'une double-enveloppe alimentée par un bain thermostaté à circulation d'huile, d'une pâle d'agitation, d'un thermomètre, d'une tête de distillation associée à un réfrigérant et à une recette de distillation, on introduit 146 g d'isosorbide (1 mole) et 222g d'acide n-octanoïque et 113g d'acide n-décanoique (c'est-à-dire 2.2 moles du mélange d'acides constitué de 70% en poids d'acide n-octanoique et 30% en poids d'acide n-décanoique pour 1 mole d'isosorbide).

Le système d'agitation est mis en fonctionnement à 400 tr/mn, ainsi que le bain thermostaté avec une consigne de 100°C. Quand la température du milieu réactionnel atteint 60°C, on ajoute 2,92 g d'acide p-toluène sulfonique (APTS) monohydrate (2 % commercial par rapport à l'isosorbide sec) et 0,90 g d'acide hypophosphoreux à 50 % soit 0,3 % en sec par rapport à l'isosorbide sec. La consigne du bain thermostaté est ensuite fixée à 150°C et l'agitation à 650 tr/mn. L'ensemble du montage est alors relié à une pompe à vide munie d'un vacuomètre dont on fixe la consigne à 100 mbar.

Lorsque la température du milieu réactionnel atteint 115°C environ, l'eau issue de la réaction d'estérification est distillée et recueillie dans la recette. Après 2 heures de réaction, la quantité d'eau distillée correspond à environ 85 % de la quantité d'eau théorique pour une réaction totale. Le vide est alors progressivement abaissé sur 3 heures supplémentaires jusqu'à 25 mbar, pendant que la température du milieu réactionnel atteint naturellement 140°C. Après 5 heures de réaction, l'eau distillée atteint 94 % de la théorie.

Le milieu réactionnel est ensuite refroidi jusqu'à 100°C environ, et on neutralise les acidités fortes de l'APTS et de l'acide hypophosphoreux par l'ajout de 1,8 g de soude à 50 %. On distille ensuite sous vide l'acide en excès qui n'a pas réagi (5 mbar ; température de vapeur : 115°C). La température du bouilleur évolue de 130 à 220°C environ pendant cette évaporation. Après refroidissement à 100°C, le produit est décoloré par un traitement au charbon actif. La composition ainsi purifiée, dénommée DEI 1, présente une quantité massique en diester d'isosorbide de 95.4 %.

**Exemple** 2 **(COMPARATIF)**

On réalise un essai 2 selon le protocole opératoire de l'essai 1 en substituant le mélange d'acides par un mélange d'acides gras constitué de 30% en poids d'acide n-octanoïque et 70% en poids d'acide n-décanoïque.

La composition finale, dénommée DEI 2, obtenue présente une quantité massique en diesters d'isosorbide de 95,8 %.

**Exemple 3 (SELON L'INVENTION)**

On réalise un essai 3 selon le protocole opératoire de l'essai 1 en substituant le mélange d'acides par un mélange d'acides gras constitué de 40% en poids d'acide n-octanoïque, 30% en poids d'acide n-décanoïque et 30% en poids d'acide laurique.

La composition finale, dénommée DEI 3, obtenue présente une quantité massique en diesters d'isosorbide de 95,1 %.

**Exemple 4 (SELON L'INVENTION)**

On réalise un essai 4 selon le protocole opératoire de l'essai 3 en introduisant 3 moles du mélange d'acides par mole d'isosorbide. Après 5 heures de réaction, l'eau distillée atteint 97% de la théorie.

La composition finale, dénommée DEI 4, obtenue présente une quantité massique en diesters d'isosorbide de 98,5 %.

**Exemple 5 (SELON L'INVENTION)**

On réalise un essai 5 selon le protocole opératoire de l'essai 4 en substituant le mélange d'acides par un mélange d'acides gras constitué de 25% en poids d'acide n-octanoïque, 45% en poids d'acide n-décanoïque et 30% en poids d'acide n-héxanoïque.

La composition finale, dénommée DEI 5, obtenue présente une quantité massique en diesters d'isosorbide de 98,2 %.

**Exemple 6 (SELON L'INVENTION)**

On réalise un essai 6 selon le protocole opératoire de l'essai 4 en substituant le mélange d'acides par un mélange d'acides gras constitué de 50% en poids d'acide n-octanoïque, 20% en poids d'acide n-décanoïque et 30% en poids d'acide myristique.

La composition finale, dénommée DEI 6, obtenue présente une quantité massique en diesters d'isosorbide de 98,2 %.

**Exemple 7 (SELON L'INVENTION)**

On réalise un essai 7 selon le protocole opératoire de l'essai 4 en substituant le mélange d'acides par un mélange d'acides gras constitué de 25% en poids d'acide n-octanoïque, 45% en poids d'acide n-décanoïque et 30% en poids d'acide linoléique.

La composition finale, dénommée DEI 7, obtenue présente une quantité massique en diesters d'isosorbide de 97,0 %.

**Exemple 8 (SELON L'INVENTION)**

On réalise un essai 8 selon le protocole opératoire de l'essai 4 en substituant le mélange d'acides par un mélange d'acides gras constitué de 20% en poids d'acide n-octanoïque, 60% en poids d'acide n-décanoïque et 20% en poids d'acide laurique.

La composition finale, dénommée DEI 8, obtenue présente une quantité massique en diesters d'isosorbide de 98,5 %.

**EXEMPLE B : EVALUATION DES CARACTERISTIQUES PHYSICO-CHIMIQUES DES COMPOSITIONS DEI 1 à DEI 4**

Les compositions DEI 1 à 4 présentent un aspect liquide à température ambiante.

Les températures de congélation et de fusion sont déterminées en mesurant la viscosité du plastifiant en fonction de la température. Elle est mesurée à l'aide d'un rhéomètre de type Physica MCR Rheometer. Pour mesurer cette évolution de la viscosité, une goutte du plastifiant est placée sur un plateau de 50 mm de diamètre et un angle de 1 ° (géométrie CP 50-1) pour une mesure des modules visqueux et élastiques en fonction de la température. Le gradient de température est de 2°C/min, le balayage en température de 20°C à -30°C, la fréquence d'oscillation de 1 Hertz et la déformation de 1 à 0,1%. La température de congélation mesurée correspond à la température de changement d'état du produit soumis à l'essai et correspond au croisement des modules visqueux et élastiques. Puis, avec les mêmes gradients, fréquence d'oscillation et déformation, en appliquant un balayage en température de -30°C à 20°C, on détermine la température de fusion qui correspond également au croisement des modules visqueux et élastiques.

On détermine également pour chacune des compositions la volatilité des compositions plastifiantes.

La détermination de la volatilité des compositions plastifiantes est effectuée par différence de pesée après un temps de séjour défini dans une étuve ventilée. Un cristallisoir est pesé précisément, et une quantité d'environ 5 g pesée précisément du produit à tester y est ajoutée. Le cristallisoir est ensuite placé à l'intérieur de l'étuve à 180°C pendant 30 min. Une fois ce temps écoulé, le cristallisoir est placé dans un dessiccateur jusqu'à refroidissement, puis pesé à

nouveau. La volatilité est alors calculée selon la formule suivante : (masse plastifiant départ - masse plastifiant après séjour en étuve) x 100/masse plastifiant départ.

Les volatilités, les températures de congélation et de fusion des compositions des essais 1 à 4 figurent dans le tableau 1.

Tableau 1

| | DEI 1 | DEI 2 | DEI 3 | DEI 4 |
|---|---|---|---|---|
| Longueur moyenne acide sur les DEI | 8,6 | 9,4 | 9,8 | 9,8 |
| Volatilité (%) | 1,10 | 0,97 | 0,86 | 0,44 |
| Température de congélation (°C) | -10 | 2 | 0 | 0 |
| Température de fusion (°C) | 4 | 22 | 11 | 13 |

La composition selon l'invention DEI 3, qui présente une pureté en DEI similaire à celle des compositions comparatives DEI 1 et DEI 2, présente une volatilité légèrement plus faible que celles de ces dernières.

Lorsque la pureté est plus élevée, comme par exemple dans le cas de la composition DEI 4, la volatilité est plus faible, ce qui s'explique logiquement par une plus faible quantité de produit plus volatils tels que les acides gras ou que les monoesters.

En ce qui concerne les compositions de DEI fabriquées à partir de mélanges binaires, plus la longueur moyenne en acide sur les DEI augmente, plus la température de congélation et de fusion augmente. Ceci est un phénomène tout à fait logique dans la mesure où les compositions de DEI obtenues après synthèse à partir d'acide n-octanoïque sont liquides à température ambiante, alors que celles obtenues après synthèse à partir d'acide n-décanoïque sont solides à température ambiante.

Cette température de fusion de 22°C pour la composition DEI 2 peut être problématique car si après stockage à basse température, par exemple à une température de 0°C, la composition est placée à température ambiante avant utilisation, cette composition peut rester dans un état solide-liquide, ce qui pose des problèmes de manipulation (impossible à pomper ou à verser), par exemple lorsque l'on souhaite l'utiliser pour la plastification de polymères.

De manière surprenante, bien que la longueur moyenne en acide sur les DEI des compositions selon l'invention soit plus élevée que celle du DEI 2, les compositions selon l'invention présentent une température de fusion plus faible. Ainsi, après stockage à basse température, la composition selon l'invention redevient liquide à température ambiante et peut être aisément utilisée. Ainsi le stockage des compositions selon l'invention est facilité.

Les compositions DEI 1 à 4 vont maintenant être utilisées pour la plastification de polymère dans les exemples 6 et 7 suivants.

**EXEMPLE C : UTILISATION DES COMPOSITIONS POUR LA PLASTIFICATION**

**DE POLYMERES**

**Exemple C-1 : Evaluation des propriétés mécaniques**

Les formulations de PVC plastifié selon l'invention sont réalisées à l'aide des produits suivants :

PVC NORVINYL S7102 (Kwert :70) : 100 parts
Stabilisant BAEROSTAB® NT 319P (Ca/Zn poudre) : 1,5 part
Co-stabilisant BAEROSTAB® LSA (huile de soja époxydée) : 2 parts
Plastifiant : 34 parts

La préparation des éprouvettes pressées destinées à la caractérisation des propriétés mécaniques se fait en plusieurs étapes.

Dans un premier temps, il est nécessaire de plastifier du PVC poudre avec la composition plastifiante dans un mélangeur planétaire de type PLANETMIX 500 (Sté Thermo Scientific) équipé d'un circuit de régulation de la température. Une masse de 500 g de PVC est introduite dans le mélangeur, avec la quantité correspondante de stabilisant thermique et de co-stabilisant thermique. Lorsque la température du mélange atteint 85°C, la composition plastifiante est incorporée sur toute la surface de la poudre de PVC. La préparation est ensuite mélangée pendant encore 8 min après l'absorption du plastifiant dans le PVC.

Dans un second temps, des plaques de PVC plastifié sont confectionnées à l'aide d'une presse de type CARVER

et d'un moule de 30 x 30 cm en inox poli miroir muni d'un cadre de 2 mm d'épaisseur et d'un couvercle inox poli miroir. Une quantité de 180 g de poudre de PVC plastifié est versé uniformément dans le cadre placé à l'intérieur du moule, puis le tout est recouvert d'un couvercle. L'ensemble est placé sur le plateau de la presse préchauffée à 185°C et le programme qui consiste en l'application d'une force de fermeture de 18 000 kg à 185°C pendant 2 min est lancé. Après refroidissement jusqu'à une température proche de 45°C, la plaque de PVC ainsi obtenue est alors démoulée.

La dernière étape consiste à découper 10 éprouvettes de type 5A (Dimensions : 25 mm x 4 mm ; épaisseur de 2 mm) à l'aide d'un emporte-pièce à partir des plaques de PVC plastifié obtenues comme décrit précédemment.

Ces éprouvettes sont ensuite caractérisées en traction sur un banc de traction ou extensomètre de type INSTRON Modèle « 5966 » avec les paramètres suivants : Vitesse d'avancement = 50 mm / min ; Cellule = 5 KN. La précontrainte est remise à zéro une fois l'éprouvette en place et les mors serrés. L'extensomètre trace la courbe contrainte/déformation de l'éprouvette jusqu'à sa rupture.

En fin d'essai, les valeurs de contrainte à 100 % de déformation et de déformation à la rupture sont relevées.

Le Tableau 2 ci-après présente, pour chaque composition testée, les valeurs de contrainte à 100 % de déformation et de déformation à la rupture obtenues.

Tableau 2

| Plastifiant | Contrainte à 100% de déformation (MPa) | Déformation ou allongement à la rupture (%) |
|---|---|---|
| DEI 1 | 15,2 | 307 |
| DEI 2 | 16,4 | 338 |
| DEI 3 | 16,2 | 307 |
| DEI 4 | 16,4 | 316 |

Ces essais montrent que les compositions selon l'invention plastifient le polymère de manière au moins aussi satisfaisante que les DEI comparatifs.

**Exemple C-2 : Effet sur les propriétés de migration de la composition**

**plastifiante**

Un des critères essentiels pour tout polymère plastifié est le taux de migration de la composition plastifiante utilisée. En effet, celui-ci doit être minimal si l'on souhaite préserver les propriétés du matériau dans le temps.

<u>Préparation des essais :</u>

A partir d'une plaque de PVC plastifié telle que réalisée dans l'exemple C-1, on découpe des éprouvettes de PVC (40 x 40 mm, épaisseur 2 mm). Elles sont conditionnées pendant 72 h à 20°C - 65 % HR. Pour chaque éprouvette de PVC à tester, deux supports absorbants de PVC non plastifié de type KOMADUR (Sté SIGMA PLV) de dimension 80 x 80 mm sur 1 mm d'épaisseur sont préparés. Les éprouvettes et les supports absorbants sont pesés sur une balance de précision. Les éprouvettes de PVC plastifiées sont ensuite placées entre les deux supports absorbants, au centre de ceux-ci. Cet ensemble est disposé entre deux plaques de verre, et un poids de 5 kg est posé encore au-dessus. Le tout est placé dans une étuve ventilée à 70°C pendant une semaine. Après une semaine d'étuve, les éprouvettes sont remises à conditionner à 20°C - 65 % HR pendant 2 jours. Enfin, elles sont pesées à nouveau afin de déterminer le taux de migration de l'éprouvette par le calcul suivant :

(masse éprouvette avant étuve – masse éprouvette après étuve) x 100 / masse éprouvette avant étuve.

Les résultats sont présentés dans le tableau 3 suivant.

Tableau 3

| Plastifiant | Taux de migration (%) | |
|---|---|---|
| DEI 1 | 2,1 | |
| DEI 2 | 1,8 | |

(suite)

| Plastifiant | Taux de migration (%) | |
|---|---|---|
| DEI 3 | 1,3 | |
| DEI 4 | 1,1 | |

Ce tableau montre que les compositions selon l'invention permettent de diminuer le taux de migration par rapport aux compositions comparatives, notamment par rapport à DEI 1.

**Exemple C-3 : Effet sur la résistance à la lixiviation de la composition plastifiante**

Préparation des essais :

A partir d'une plaque de PVC plastifié telle que réalisée dans l'exemple C-1, on découpe des éprouvettes de PVC (50 x 50 mm, épaisseur 2 mm). Elles sont conditionnées pendant 48 h à 20°C - 65 % HR. Chaque éprouvetteest pesée puis immergée séparément dans 190 g d'eau déminéralisée à l'intérieur d'un pot twist-off fermé hermétiquement. Le tout est mis à l'étuve à 70°C pour 8 semaines. Après 8 semaines d'étuve, les éprouvettes sont sorties et essuyées dans du papier absorbant. Les éprouvettes vont ensuite subir un séchage en étuve à vide à 53°C pendant 24 h avant d'être à nouveau pesées.

La perte de masse par lixiviation est alors déterminée par le calcul suivant : (masse éprouvette avant étuve - masse éprouvette après séchage à vide) x 100/masse éprouvette avant étuve.

Les résultats sont présentés dans le tableau 4 suivant.

Tableau 4

| Plastifiant | Perte de masse de l'échantillon par lixiviation (%) |
|---|---|
| DEI 1 | -0,40% |
| DEI 2 | -0,35% |
| DEI 3 | -0,28% |
| DEI 4 | -0,15% |

Ce tableau montre que les compositions selon l'invention permettent d'améliorer la résistance à la lixiviation par rapport aux compositions comparatives, notamment par rapport à DEI 1.

## Revendications

1. Procédé de fabrication d'une composition de diesters de 1,4:3,6-dianydrohexitol, **caractérisé en ce qu'**il comprend une étape d'estérification de 1,4 : 3,6-dianhydrohexitol par une composition d'acides carboxyliques, ladite composition d'acides carboxyliques comprenant :

    • au moins un acide de formule $C7H15COOH$ (X) et un acide de formule $C9H19COOH$ (Y), le ratio massique (X)/(Y) allant de 10/90 à 90/10 ;
    • au moins un acide de formule différente de (X) et (Y) choisi parmi l'acide hexanoïque, l'acide laurique, l'acide myristique, l'acide oléïque ou l'acide linoléique.

2. Procédé de fabrication selon la revendication précédente, **caractérisé en ce que** la composition d'acides carboxyliques comprend, par rapport à sa masse totale, au moins 50% en masse des acides (X) et (Y), avantageusement au moins 65%.

3. Procédé de fabrication selon l'une ou l'autre des revendications 1 et 2, **caractérisé en ce que** le ratio massique (X)/(Y) va de 15/85 à 75/25.

4. Procédé de fabrication selon la revendication 3, **caractérisé en ce que** le ratio massique (X)/(Y) va de 20/80 à 65/35.

**5.** Procédé de fabrication selon la revendication 4, **caractérisé en ce que** le ratio massique (X)/(Y) va de 25/75 à 55/45.

**6.** Procédé de fabrication selon l'une quelconque des revendications précédentes, **caractérisé en ce que** l'acide de formule différente de (X) et (Y) est l'acide laurique.

**7.** Procédé de fabrication selon l'une quelconque des revendications précédentes, **caractérisé en ce que** le 1,4:3,6-dianhydrohexitol est choisi parmi l'isomannide, l'isoidide, l'isosorbide ou leurs mélanges, préférentiellement l'isosorbide.

**8.** Composition de diesters de 1,4:3,6-dianhydrohexitols susceptible d'être obtenue par le procédé selon l'une quelconque des revendications 1 à 7.

**9.** Utilisation de la composition selon la revendication 8 pour la plastification de polymères.

**10.** Utilisation selon la revendication 9, **caractérisée en ce que** le polymère est choisi parmi les polymères vinyliques tels que le polychlorure de vinyle et les copolymères de chlorure de vinyle, les polyuréthanes, les polyesters, les polymères cellulosiques, les amidons, les polymères acryliques, les polyacétates, les caoutchoucs naturels ou synthétiques, les polyamides et les mélanges de ces polymères, préférentiellement le polychlorure de vinyle.

## Patentansprüche

**1.** Verfahren zur Herstellung einer Zusammensetzung aus Diestern von 1,4:3,6-Dianhydrohexitol, **dadurch gekennzeichnet, dass** es einen Veresterungsschritt von 1,4:3,6-Dianhydrohexitol durch eine Zusammensetzung aus Carbonsäuren enthält, wobei besagte Zusammensetzung aus Carbonsäuren enthält:

- mindestens eine Säure der Formel $C7H15COOH$ (X) und eine Säure der Formel $C9H19COOH$ (Y), wobei das Gewichtsverhältnis (X)/(Y) im Bereich von 10/90 bis 90/10 liegt;
- mindestens eine Säure einer Formel die sich von (X) und (Y) unterscheidet und ausgewählt ist aus Hexansäure, Laurinsäure, Myristinsäure, Ölsäure oder Linolsäure.

**2.** Verfahren zur Herstellung nach dem vorausgehenden Anspruch, **dadurch gekennzeichnet, dass** die Zusammensetzung aus Carbonsäuren, bezogen auf ihre Gesamtmasse, mindestens 50 Gew.-% an Säuren (X) und (Y), vorzugsweise mindestens 65 Gew.-%, enthält.

**3.** Verfahren zur Herstellung nach einem der beiden Ansprüche 1 und 2, **dadurch gekennzeichnet, dass** das Gewichtsverhältnis (X)/(Y) im Bereich von 15/85 bis 75/25 liegt.

**4.** Verfahren zur Herstellung nach Anspruch 3, **dadurch gekennzeichnet, dass** das Gewichtsverhältnis (X)/(Y) im Bereich von 20/80 bis 65/35 liegt.

**5.** Verfahren zur Herstellung nach Anspruch 4, **dadurch gekennzeichnet, dass** das Gewichtsverhältnis (X)/(Y) im Bereich von 25/75 bis 55/45 liegt.

**6.** Verfahren zur Herstellung nach irgendeinem der vorausgehenden Ansprüche, **dadurch gekennzeichnet, dass** die Säure der Formel, die sich von (X) und (Y) unterscheidet, Laurinsäure ist.

**7.** Verfahren zur Herstellung nach irgendeinem der vorausgehenden Ansprüche, **dadurch gekennzeichnet, dass** das 1,4:3,6-Dianhydrohexitol ausgewählt ist aus Isomannid, Isoidid, Isosorbid oder deren Mischungen, vorzugsweise Isosorbid.

**8.** Zusammensetzung aus Diestern von 1,4:3,6-Dianhydrohexitolen, herstellbar durch ein Verfahren nach irgendeinem der Ansprüche 1 bis 7.

**9.** Verwendung der Zusammensetzung nach Anspruch 8 zur Polymerplastifizierung.

**10.** Verwendung nach Anspruch 9, **dadurch gekennzeichnet, dass** das Polymer ausgewählt ist aus Vinylpolymeren wie Polyvinylchlorid und Co-Polymeren von Vinylchlorid, Polyurethanen, Polyestern, Zellulosen, Stärken, Polyacrylaten, Polyacetaten, natürlichen oder synthetischen Kautschuken, Polyamiden und Mischungen dieser Polymere, vorzugsweise Polyvinylchlorid.

## Claims

1. A process for manufacturing a 1,4:3,6-dianydrohexitol diester composition, **characterized in that** it comprises a step of esterifying 1,4:3,6-dianhydrohexitol with a carboxylic acid composition, said carboxylic acid composition comprising:

    • at least one acid of formula $C_7H_{15}COOH$ (X) and an acid of formula $C_9H_{19}COOH$ (Y), the mass ratio (X)/(Y) ranging from 10/90 to 90/10;
    • at least one acid of formula different from (X) and (Y) chosen from hexanoic acid, lauric acid, myristic acid, oleic acid and linoleic acid.

2. The manufacturing process as claimed in the preceding claim, **characterized in that** the carboxylic acid composition comprises, relative to its total mass, at least 50% by mass of acids (X) and (Y), advantageously at least 65%.

3. The manufacturing process as claimed in either of claims 1 and 2, **characterized in that** the mass ratio (X)/(Y) ranges from 15/85 to 75/25.

4. The manufacturing process as claimed in claim 3, **characterized in that** the mass ratio (X)/(Y) ranges from 20/80 to 65/35.

5. The manufacturing process as claimed in claim 4, **characterized in that** the mass ratio (X)/(Y) ranges from 25/75 to 55/45.

6. The manufacturing process as claimed in any one of the preceding claims, **characterized in that** the acid of formula other than (X) and (Y) is lauric acid.

7. The manufacturing process as claimed in any one of the preceding claims, **characterized in that** the 1,4:3,6-dianhydrohexitol is chosen from isomannide, isoidide and isosorbide or mixtures thereof, preferentially isosorbide.

8. A 1,4:3,6-dianhydrohexitol diester composition which may be obtained via the process as claimed in any one of claims 1 to 7.

9. Use of the composition as claimed in claim 8 for plasticizing polymers.

10. The use as claimed in claim 9, **characterized in that** the polymer is chosen from vinyl polymers such as polyvinyl chloride and vinyl chloride copolymers, polyurethanes, polyesters, cellulose-based polymers, starches, acrylic polymers, polyacetates, natural or synthetic rubbers, polyamides, and mixtures of these polymers, preferentially polyvinyl chloride.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 0078853 A
- WO 9932427 A
- WO 9945060 A
- WO 2008095571 A1
- US 2387842 A
- WO 2013092655 A
- WO 2013092649 A
- WO 9945060 A1
- WO 2006103338 A1


### Littérature non-brevet citée dans la description


- **HACHIHAMA et al.** *Technology reports of the Osaka University,* 1953, vol. 3, 191-200